# EUROPEAN PATENT SPECIFICATION

(11) **EP 4 529 941 B1**
(45) Date of publication and mention of the grant of the patent: **24.06.2026**
(21) Application number: 24189783.4
(22) Date of filing: 19.07.2024
(51) Int. Cl.: A61M 25/00, A61M 25/10

(54) **CATHETER BALLOON**
KATHETERBALLON
BALLONNET DE CATHÉTER

(30) Priority: 27.09.2023 JP 2023164642
(43) Date of publication of application: 02.04.2025
(73) Proprietor: Terumo Kabushiki Kaisha, Tokyo 151-0072 (JP)
(72) Inventor: ITO, Keisuke, Fujinomiya-shi, 418-0015 (JP); KUWANO, Yoichiro, Somerset, 08873 (US); SHIMOJI, Erena, Nakai-machi, 259-0151 (JP)
(74) Representative: Casalonga

(56) References cited:
- EP-A1- 1 611 917
- US-A- 5 447 497
- US-A1- 2013 237 950
- US-A1- 2015 290 434

## Description

### TECHNOLOGICAL FIELD

The present invention generally relates to a catheter balloon.

### BACKGROUND DISCUSSION

A balloon catheter is widely known as a medical device that dilates a stenosis (lesion) formed in a living body lumen such as a blood vessel. A balloon included in the balloon catheter may be devised in various ways in order to improve a therapeutic effect. For example, one type of performance required for the balloon is pressure resistance (fracture resistance) of the balloon.

Generally, the balloon is manufactured by molding a cylindrical member (balloon material) including a predetermined resin material into a desired shape by a molding method such as blow molding. Thus, performance derived from physical properties of the balloon depends on a material of the balloon material used for the balloon. In view of such a point, for example, JP 2015-181772 A proposes a balloon catheter including a balloon including two layers having different physical properties.

### SUMMARY

In the balloon catheter of JP 2015-181772 A, by adopting a balloon having a multilayer structure including a shape memory layer having a predetermined glass transition temperature and a base material layer having a higher strength (rupture strength) than the shape memory layer, a shape restoring property of the balloon after dilation is improved. However, the balloon catheter described in JP 2015-181772 A has the following problem.

In the balloon having the multilayer structure adopted in JP 2015-181772 A, the strength of the base material layer is higher than the strength of the shape memory layer. As described above, since the layers have different strengths, in a case where rupture (cracking) occurs in the shape memory layer having low strength, the balloon is substantially configured only by the base material layer. When supply of fluid to the balloon is continued in such a state, a high pressure is applied only to the base material layer, and thus the balloon may be excessively dilated beyond an appropriate dilation diameter.

The catheter balloon disclosed here is capable of preventing a balloon having a multilayer structure from being excessively dilated beyond an appropriate dilation diameter due to rupture of any one layer more easily than other layers in the balloon.

Since the catheter balloon described above has a multilayer structure including the first layer and the second layer disposed adjacent to each other in the thickness direction of the balloon, pressure resistance of the balloon can be effectively enhanced. Furthermore, in the catheter balloon described above, the rupture strength of the first layer and the rupture strength of the second layer are the same, and thus it is possible to prevent the balloon from being excessively dilated beyond an appropriate dilation diameter due to rupture of any one layer more easily than other layers during a procedure. Furthermore, in the catheter balloon described above, the first layer and the second layer are configured by different materials, and thus the balloon can have desired physical properties by selecting an optional different material as a material of each layer.

Another aspect involves a catheter balloon connected to a shaft portion and having an interior configured to receive a fluid to expand the catheter balloon. The catheter balloon comprises a distal end portion and a proximal end portion at opposite ends of the catheter balloon, with the distal end of the catheter balloon being fixed to the shaft portion and the proximal end of the catheter balloon being fixed to the shaft so that an interior of the catheter balloon forms a space configured to receive a fluid to expand the catheter balloon. The catheter balloon also comprises a first layer made of a material and a second layer made of a material, with the material from which the first layer is made being different from the material from which the second layer is made. The second layer is positioned in covering relation to the first layer, the first and second layers possess a rupture strength, and the rupture strength of the first layer is the same as the rupture strength of the second layer. In the description certain references are made to non-SI unit (atm). These are to be translated to corresponding SI unit Pascal (Pa) using the factor: 1 atm = approximately 101,325 Pa.

### BRIEF DESCRIPTION OF THE DRAWINGS

FIG. 1 is a view illustrating a balloon catheter according to an embodiment.
FIG. 2 is an enlarged cross-sectional view illustrating a vicinity of a distal end of the balloon catheter.
FIG. 3 is a cross-sectional view (axially orthogonal cross-sectional view) of a portion taken along the section line 3A-3A in FIG. 2.
FIG. 4 is a cross-sectional view (axially orthogonal cross-sectional view) of a catheter balloon according to a modification.

### DETAILED DESCRIPTION

Hereinafter, an embodiment and modification of the balloon catheter disclosed here will be described with reference to the drawings, it being understood that the embodiment and modification described represent examples of the new balloon catheter disclosed here.

Dimensional ratios in the drawings (for example, dimensional ratios of a thickness of each layer of a balloon to other members) are exaggerated for convenience of description, and may be different from actual ratios.

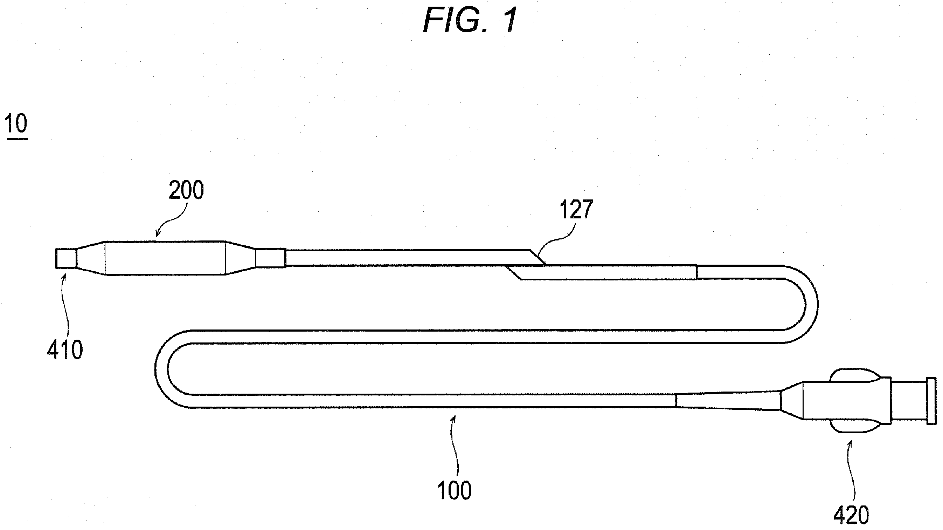

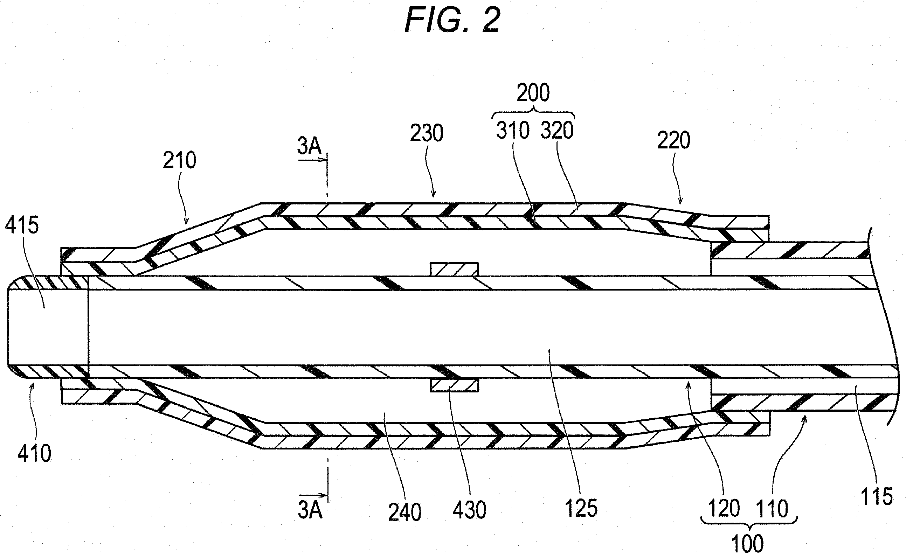

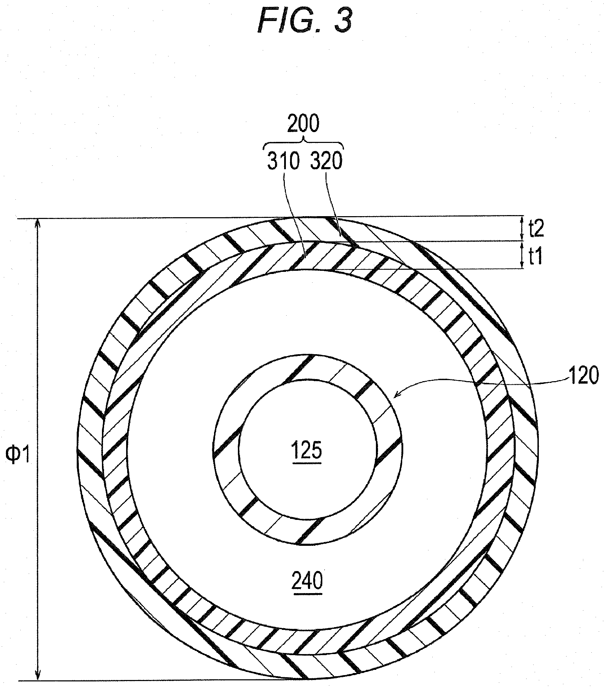

FIG. 1 is a view illustrating a balloon catheter 10 according to an embodiment, FIG. 2 is a partial cross-sectional view of a vicinity of a distal end of the balloon catheter 10, and FIG. 3 is an axially orthogonal cross-sectional view taken along the section line 3A-3A illustrated in FIG. 2.

In the present embodiment, the new catheter balloon will be described through the balloon catheter 10. Each part of the balloon catheter 10 can optionally adopt a structure known in a medical field, and is not limited only to a configuration described below. For example, the catheter balloon can also be applied to a balloon or the like of a stent delivery catheter.

### <Balloon Catheter 10>

As illustrated in FIG. 1, the balloon catheter 10 includes a shaft portion 100, a catheter balloon 200 that can be dilated and contracted with injection and discharge of a pressurizing medium, a distal end tip 410, and a hub portion 420.

In the present specification, a side to be introduced into a living body (a side on which the distal end tip 410 is disposed) in the balloon catheter 10 is referred to as a distal end side, and an end positioned on the distal end side in each member and component is referred to as a "distal end". Furthermore, a side on which the hub portion 420 is disposed in the balloon catheter 10 is referred to as a proximal end side, and an end positioned on the proximal end side in each member and component is referred to as a "proximal end". Furthermore, a direction from the distal end to the proximal end (or from the proximal end to the distal end) is referred to as an "axial direction (longitudinal direction)". The definitions of the distal end side (distal end), the proximal end side (proximal end), and the axial direction described above are similar for a position and direction of each end of the catheter balloon 200.

The distal end tip 410 is disposed or provided for the purpose of preventing damage to a biological organ (an inner wall of a blood vessel, or the like) when the distal end of the balloon catheter 10 comes into contact with the biological organ.

As illustrated in FIG. 2, the distal end tip 410 is disposed at a distal end of the shaft portion 100. A lumen 415 through which a guide wire (not illustrated) can be inserted is formed inside the distal end tip 410.

The distal end tip 410 can be configured by, for example, a more flexible material than the shaft portion 100. The distal end tip 410 can be formed of, for example, a polymer material such as polyolefins (for example, polyethylene, polypropylene, polybutene, ethylene-propylene copolymer, ethylene-vinyl acetate copolymer, ionomer, or a mixture of two or more types thereof), polyvinyl chloride, polyamide, polyamide elastomer, polyurethane, polyurethane elastomer, polyimide, or fluororesin, or a mixture thereof, or a multilayer tube of two or more types of the polymer materials described above.

As illustrated in FIGS. 1 and 2, the catheter balloon 200 is fixed in a vicinity of the distal end of the shaft portion 100. At a proximal end of the shaft portion 100, the hub portion 420 to which an indeflator for supplying various pressurizing media to the catheter balloon 200 and discharging the pressurizing media from the catheter balloon 200 can be connected is disposed.

As illustrated in FIG. 2, the shaft portion 100 includes an outer shaft 110 and an inner shaft 120. The inner shaft 120 is inserted through a lumen 115 of the outer shaft 110.

A distal end of the inner shaft 120 protrudes more toward the distal end side than the outer shaft 110 by a predetermined length. The catheter balloon 200 has a distal end fixed to the distal end of the inner shaft 120 and a proximal end fixed to a distal end of the outer shaft 110.

The lumen 115 of the outer shaft 110 and the inner shaft 120 form a dilation lumen communicating with a space portion 240 of the catheter balloon 200.

As illustrated in FIG. 1, a proximal end of the inner shaft 120 forms a guide wire port 127 serving as an inlet/outlet port for inserting the guide wire into the shaft portion 100. A lumen 125 of the inner shaft 120 forms a guide wire lumen through which the guide wire can be inserted. That is, the balloon catheter 10 is configured as a so-called rapid exchange type catheter. The balloon catheter 10 may be configured as a so-called over-the-wire type catheter.

On the shaft portion 100, for example, a contrast marker 430 for indicating a center position in the axial direction of a straight portion 230 of the catheter balloon 200 can be disposed. The contrast marker 430 can be configured by, for example, a metal such as platinum, gold, silver, iridium, titanium, or tungsten, or an alloy thereof.

The outer shaft 110 and the inner shaft 120 can be formed of, for example, polyolefins such as polyethylene, polypropylene, ethylene-propylene copolymer, and ethylene-vinyl acetate copolymer, thermoplastic resins such as soft polyvinyl chloride, various elastomers such as polyurethane elastomers, polyamide elastomers, and polyester elastomers, and crystalline plastics such as polyamide, crystalline polyethylene, and crystalline polypropylene.

### <Catheter Balloon 200>

As illustrated in FIG. 2, the catheter balloon 200 includes a distal end side tapered portion 210 having a cross-sectional shape inclined in a direction approaching a central axis of the shaft portion 100 toward the distal end side, a proximal end side tapered portion 220 having a cross-sectional shape inclined in a direction approaching the central axis of the shaft portion 100 toward the proximal end side, and the straight portion 230 disposed between the distal end side tapered portion 210 and the proximal end side tapered portion 220. FIG. 2 illustrates the catheter balloon 200 in a dilated state for convenience of briefly describing the catheter balloon.

The catheter balloon 200 is fixed to the shaft portion 100 so as to define the space portion 240 into which a pressurizing medium (for example, fluid such as saline or a contrast medium) can be injected between the catheter balloon 200 and the inner shaft 120.

As illustrated in FIGS. 2 and 3, the catheter balloon 200 has a first layer 310 and a second layer 320 disposed adjacent to each other in a thickness direction of the balloon 200.

In the present embodiment, the first layer 310 and the second layer 320 are configured by different materials. Furthermore, a rupture strength of the first layer 310 and a rupture strength of the second layer 320 are the same.

The rupture strength of the first layer 310 and the rupture strength of the second layer 320 are, for example, 40 to 62 MPa. The "rupture strength" in the present specification is a value (tensile strength/rupture point) defined on the basis of a tensile test defined by any one of ISO 527, ASTM D 638, and JIS K 7311.

Furthermore, the phrase "disposed adjacent to each other in a thickness direction of the balloon 200" described above means a state where the first layer 310 and the second layer 320 are disposed adjacent to each other in a state where an inner surface of the second layer 320 is in contact with (abutting) an outer surface of the first layer 310.

As illustrated in FIGS. 2 and 3, the second layer 320 is disposed so as to cover the first layer 310. That is, the first layer 310 is an inner layer constituting an inner surface of the catheter balloon 200, and the second layer 320 is an outer layer constituting an outer surface of the catheter balloon 200.

For example, an elongation percentage of the first layer 310 can be configured to be larger than an elongation percentage of the second layer 320.

The elongation percentage of the first layer 310 is, for example, 360 to 700%. Furthermore, the elongation percentage of the second layer 320 is, for example, 50 to 360%. The "elongation percentage" in the present specification is a value defined on the basis of a tensile test defined by any one of ISO 527, ASTM D 638, and JIS K 7311.

For example, the hardness of the first layer 310 can be configured to be smaller than the hardness of the second layer 320.

The hardness of the first layer 310 is, for example, 62 to 78 Shore D. Furthermore, the hardness of the second layer 320 is, for example, 52 to 70 Shore D. The "hardness" in the present specification is a value defined on the basis of a hardness test defined by any one of ISO 868, ASTM D 785, and JIS K 7311.

For example, a tensile modulus of elasticity of the first layer 310 can be configured to be smaller than a tensile modulus of elasticity of the second layer 320.

The tensile modulus of elasticity of the first layer 310 is, for example, 460 MPa. Furthermore, the tensile modulus of elasticity of the second layer 320 is, for example, 1100 to 2700 MPa. The "tensile modulus of elasticity" in the present specification is a value defined on the basis of a tensile test defined by ISO 527 or ASTM D 638.

The first layer 310 can be configured by, for example, any one of a polyamide elastomer, a polyurethane, and a polyurethane elastomer. That is, by way of example, the first layer 310 may be fabricated from any one of a polyamide elastomer, a polyurethane, and a polyurethane elastomer.

The second layer 320 can be configured by, for example, a polyamide. That is, by way of example, the second layer 320 may be fabricated from a polyamide.

A suitable combination of materials of the first layer 310 and the second layer 320 is exemplified below. However, the materials used for the first layer 310 and the second layer 320 are not limited only to the content described below by way of example.

For the first layer 310, for example, a polyamide elastomer (product name ELG6260 manufactured by EMS-CHEMIE (Japan) Ltd.) having a rupture strength of 50 MPa (ISO 527), a tensile modulus of elasticity of 460 MPa (ISO 527), an elongation percentage (rupture point) of 400% (ISO 527), and 62 Shore D (ISO 868) can be suitably used. In a case where the material described above is used for the first layer 310, for the second layer 320, a polyamide (product name L25 manufactured by EMS-CHEMIE (Japan) Ltd.) having a rupture strength of 50 MPa (ISO 527), a tensile modulus of elasticity of 1100 MPa (ISO 527), an elongation percentage (rupture point) of > 50% (ISO 527), and 70 Shore D (ISO 868) can be suitably used.

Furthermore, for the first layer 310, for example, a polyamide elastomer (product name Pebax (registered trademark) manufactured by ARKEMA K.K.) having a rupture strength of 62 MPa (ASTM D 638), an elongation percentage (rupture point) of 360% (ASTM D 638), and 72 Shore D (ISO 868) can be suitably used.

In a case where the material described above is used for the first layer 310, for the second layer 320, polyamide (Mitsuboshi Belting Ltd., Nylon6) having a rupture strength of 62 MPa (ISO 527), a tensile modulus of elasticity of 2700 MPa (ASTM D 638), an elongation percentage (rupture point) of 290% (ASTM D 638), and 52 Shore D (ASTM D 785) can be suitably used.

Furthermore, for the first layer 310, for example, a polyurethane elastomer (product name Elastollan (registered trademark) manufactured by BASF SE) having a rupture strength of 40 MPa (JIS K 7311), an elongation percentage (rupture point) of 700% (JIS K 7311), and 72 ± 3 Shore D (JIS K 7311) can be suitably used. In a case where the material described above is used for the first layer 310, for the second layer 320, a polyamide (product name L20LF manufactured by EMS-CHEMIE (Japan) Ltd.) having a rupture strength of 50 MPa (ISO 527), a tensile modulus of elasticity of 1100 MPa (ISO 527), an elongation percentage (rupture point) of > 50% (JIS K 7311), and 52 Shore D (ISO 868) can be suitably used.

An outer diameter φ1 (outer diameter in an undilated state after molding) of the catheter balloon 200 can be, for example, 3.0 to 7.0 mm.

A film thickness t1 of the first layer 310 can be, for example, 0.5 to 50.0 µm.

A film thickness t2 of the second layer 320 can be, for example, 0.5 to 50.0 µm.

Hereinafter, operation and effects of the present embodiment will be described.

The catheter balloon 200 according to the present embodiment includes the first layer 310 and the second layer 320 disposed adjacent to each other in the thickness direction of the balloon 200, the first layer 310 and the second layer 320 are configured by different materials, and the rupture strength of the first layer 310 and the rupture strength of the second layer 320 are the same.

Since the catheter balloon 200 configured as described above has a multilayer structure including the first layer 310 and the second layer 320 disposed adjacent to each other in the thickness direction of the balloon 200, pressure resistance of the balloon 200 can be effectively enhanced. Furthermore, in the catheter balloon 200, the rupture strength of the first layer 310 and the rupture strength of the second layer 320 are the same, and thus it is possible to prevent the balloon 200 from being excessively dilated beyond an appropriate dilation diameter due to rupture of any one layer more easily than the other layer (other layers) during a procedure. Furthermore, in the catheter balloon 200, the first layer 310 and the second layer 320 are configured by different materials, and thus the balloon 200 can have desired physical properties by selecting an optional different material as a material of each of the layers 310 and 320.

Furthermore, the second layer 320 is disposed so as to cover the first layer 310, and the elongation percentage of the first layer 310 can be configured to be larger than the elongation percentage of the second layer 320.

In the catheter balloon 200 configured as described above, when fluid is supplied into the space portion 240, dilation starts from the first layer 310 disposed inside the second layer 320. Since the elongation percentage of the first layer 310 is larger than the elongation percentage of the second layer 320, after the first layer 310 is dilated to a predetermined outer diameter, dilation of the second layer 320 positioned on an outer surface side of the first layer 310 is started so as to follow the dilation of the first layer 310. Thus, when the fluid is supplied into the space portion 240, the dilation of the catheter balloon 200 proceeds in the order of the first layer 310 and the second layer 320, and can be smoothly dilated to a desired dilation diameter.

Furthermore, the hardness of the first layer 310 can be configured to be smaller than the hardness of the second layer 320.

In the catheter balloon 200 configured as described above, when the first layer 310 is dilated, a dilation diameter of the first layer 310 is controlled by the second layer 320 disposed on the outer surface side of the first layer 310. Thus, in the catheter balloon 200, it is possible to prevent excessive dilation of the balloon 200 due to excessive dilation of the first layer 310 having the larger elongation percentage than the second layer 320.

Furthermore, the tensile modulus of elasticity of the first layer 310 can be configured to be smaller than the tensile modulus of elasticity of the second layer 320.

In the catheter balloon 200 configured as described above, the tensile modulus of elasticity of the second layer 320 is larger than the tensile modulus of elasticity of the first layer 310, and thus, when fluid flows into the space portion 240 and the same pressurizing force is applied to the first layer 310 and the second layer 320, the second layer 320 is less likely to be deformed than the first layer 310. Thus, the catheter balloon 200 can be prevented from being excessively dilated by the second layer 320. Furthermore, since the second layer 320 is less likely to be deformed than the first layer 310, the second layer 320 is less likely to be elongated than the first layer 310 in a state where the same pressurizing force is applied. Thus, the second layer 320 can also contribute to improvement in pressure resistance of the catheter balloon 200.

Furthermore, the first layer 310 can be configured by any one of a polyamide elastomer, a polyurethane, and a polyurethane elastomer, and the second layer 320 can be configured by a polyamide.

The catheter balloon 200 configured as described above can effectively enhance the pressure resistance of the balloon 200 while making the rupture strength of the first layer 310 and the rupture strength of the second layer 320 the same.

Next, a modification of the embodiment described above will be described. In the description below, features and aspects of the catheter balloon that are the same or similar to those described above are identified by the same reference numerals and a detailed description of such features is not repeated. Furthermore, content that is not particularly described can be configured similarly to the embodiment described above.

### <Modification>

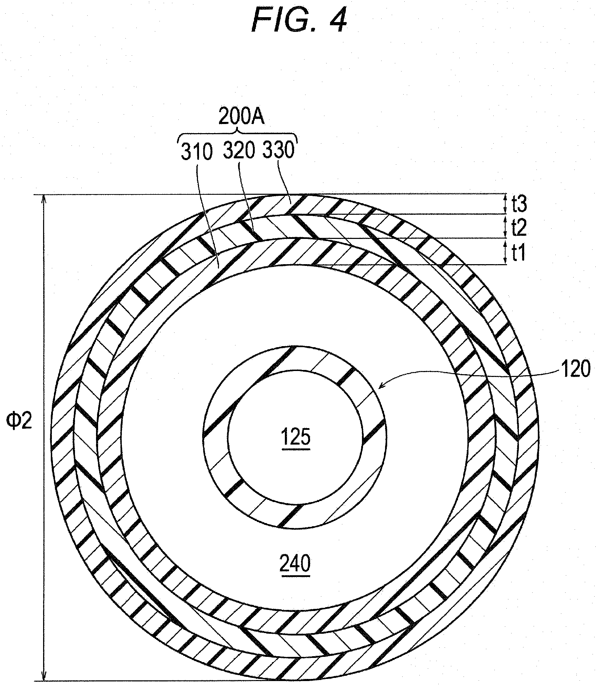

FIG. 4 illustrates a cross-sectional view (axially orthogonal cross-sectional view corresponding to FIG. 3) of a catheter balloon 200A according to the modification.

As illustrated in FIG. 4, the catheter balloon 200A includes a third layer 330 disposed adjacent to the second layer 320 so as to cover the second layer 320.

The catheter balloon 200 has a multilayer structure including three layers, namely the first layer 310, the second layer 320, and the third layer 330. The first layer 310 constitutes an inner layer (innermost layer) of the catheter balloon 200A, the second layer 320 disposed to cover the first layer 310 constitutes a middle layer of the catheter balloon 200A, and the third layer 330 disposed to cover the second layer 320 constitutes an outer layer (outermost layer) of the catheter balloon 200A.

A rupture strength of the third layer 330 is the same as the rupture strength of the first layer 310 and the rupture strength of the second layer 320.

In a case where the rupture strengths of the first layer 310 and the second layer 320 are 40 to 62 MPa as described above, the rupture strength of the third layer 330 can be 40 to 62 MPa similarly to these.

Furthermore, for example, the elongation percentage of the first layer 310 and an elongation percentage of the third layer 330 can be configured to be the same. Furthermore, for example, the elongation percentage of the first layer 310 and the elongation percentage of the third layer 330 can be configured to be larger than the elongation percentage of the second layer 320.

In a case where the elongation percentage of the first layer 310 is 360 to 700% and the elongation percentage of the second layer 320 is 50 to 360% as described above, the elongation percentage of the third layer 330 can be 360 to 700% similarly to the first layer 310.

Furthermore, for example, the hardness of the first layer 310 and hardness of the third layer 330 can be configured to be the same. Furthermore, the hardness of the first layer 310 and the hardness of the third layer 330 can be configured to be smaller than the hardness of the second layer 320.

In a case where the hardness of the first layer 310 is 62 to 78 Shore D and the hardness of the second layer 320 is 52 to 70 Shore D as described above, the hardness of the third layer 330 can be 62 to 78 Shore D similarly to the first layer 310.

Furthermore, for example, the tensile modulus of elasticity of the first layer 310 and a tensile modulus of elasticity of the third layer 330 can be configured to be the same. Furthermore, for example, the tensile modulus of elasticity of the first layer 310 and the tensile modulus of elasticity of the third layer 330 can be configured to be smaller than the tensile modulus of elasticity of the second layer 320.

In a case where the tensile modulus of elasticity of the first layer 310 is 460 MPa and the tensile modulus of elasticity of the second layer 320 is 1100 to 2700 MPa as described above, the tensile modulus of elasticity of the third layer 330 can be 460 MPa similarly to the first layer 310.

The first layer 310 can be configured by (i.e., fabricated from), for example, a polyamide elastomer.

The second layer 320 can be configured by (i.e., fabricated from), for example, a polyamide.

The third layer 330 can be configured by (i.e., fabricated from), for example, a polyamide elastomer.

An outer diameter φ2 (outer diameter in an undilated state after molding) of the catheter balloon 200A can be, for example, 4.0 to 8.0 mm.

The film thickness t1 of the first layer 310 can be, for example, 0.5 to 50.0 µm.

The film thickness t2 of the second layer 320 can be, for example, 0.5 to 50.0 µm.

A film thickness t3 of the third layer 330 can be, for example, 0.5 to 50.0 µm.

Hereinafter, operation and effects of the present modification will be described.

The catheter balloon 200A according to the present modification includes the third layer 330 disposed adjacent to the second layer 320 so as to cover the second layer 320. Furthermore, the rupture strength of the third layer 330 is the same as the rupture strength of the first layer 310 and the rupture strength of the second layer 320.

Since the catheter balloon 200A configured as described above has a multilayer structure including the three layers of the first layer 310, the second layer 320, and the third layer 330, pressure resistance of the balloon 200A can be more effectively enhanced. Furthermore, in the catheter balloon 200A, the rupture strength of the first layer 310, the rupture strength of the second layer 320, and the rupture strength of the third layer 330 are the same, and thus it is possible to prevent the balloon 200A from being excessively dilated beyond an appropriate dilation diameter due to rupture of any one layer more easily than other layers during a procedure.

Furthermore, the elongation percentage of the first layer 310 and the elongation percentage of the third layer 330 can be configured to be the same, and the elongation percentage of the first layer 310 and the elongation percentage of the third layer 330 can be configured to be larger than the elongation percentage of the second layer 320.

In the catheter balloon 200A configured as described above, when fluid is supplied into the space portion 240, dilation starts from the first layer 310 disposed inside the second layer 320. Since the elongation percentage of the first layer 310 is larger than the elongation percentage of the second layer 320, after the first layer 310 is dilated to a predetermined outer diameter, dilation of the second layer 320 positioned on an outer surface side of the first layer 310 is started so as to follow the dilation of the first layer 310. Furthermore, since the elongation percentage of the third layer 330 is larger than the elongation percentage of the second layer 320, after the second layer 320 is dilated to a predetermined outer diameter, the third layer 330 is rapidly dilated so as to follow the dilation of the second layer 320. Therefore, the catheter balloon 200A can be smoothly dilated with a pressurizing force of the fluid flowing into the space portion 240.

Furthermore, the hardness of the first layer 310 and the hardness of the third layer 330 can be configured to be the same, and the hardness of the first layer 310 and the hardness of the third layer 330 can be configured to be smaller than the hardness of the second layer 320.

In the catheter balloon 200A configured as described above, when the first layer 310 is dilated, a dilation diameter of the first layer 310 is controlled by the second layer 320 disposed on the outer surface side of the first layer 310. Thus, in the catheter balloon 200A, it is possible to prevent excessive dilation of the balloon 200A due to excessive dilation of the first layer 310 having the larger elongation percentage than the second layer 320. Furthermore, since the hardness of the second layer 320 is larger than the hardness of the third layer 330 disposed on an outer surface side of the second layer 320, the second layer 320 functions as a buffer layer that suppresses excessive dilation of the catheter balloon 200A between the first layer 310 and the third layer 330. Therefore, the catheter balloon 200A can achieve both smooth dilation and prevention of excessive dilation.

Furthermore, the tensile modulus of elasticity of the first layer 310 and the tensile modulus of elasticity of the third layer 330 can be configured to be the same, and the tensile modulus of elasticity of the first layer 310 and the tensile modulus of elasticity of the third layer 330 can be configured to be smaller than the tensile modulus of elasticity of the second layer 320.

In the catheter balloon 200A configured as described above, the tensile modulus of elasticity of the second layer 320 is larger than the tensile modulus of elasticity of the first layer 310, and thus, when fluid flows into the space portion 240 and the same pressurizing force is applied to the first layer 310 and the second layer 320, the second layer 320 is less likely to be deformed than the first layer 310. Thus, the catheter balloon 200A can be prevented from being excessively dilated by the second layer 320. Furthermore, since the second layer 320 is less likely to be deformed than the first layer 310, the second layer 320 is less likely to be elongated than the first layer 310 in a state where the same pressurizing force is applied. Thus, the second layer 320 can also contribute to improvement in pressure resistance of the catheter balloon 200A. Furthermore, since the tensile modulus of elasticity of the third layer 330 is smaller than the tensile modulus of elasticity of the second layer 320 disposed on an inner surface side of the third layer 330, when the second layer 320 is dilated, the third layer 330 can be rapidly dilated so as to follow the dilation of the second layer 320. Therefore, the catheter balloon 200A can implement improvement in pressure resistance, smooth dilation, and prevention of excessive dilation.

Furthermore, the first layer 310 can be configured by a polyamide elastomer, the second layer 320 can be configured by a polyamide, and the third layer 330 can be configured by a polyamide elastomer.

The catheter balloon 200A configured as described above can effectively enhance the pressure resistance of the balloon 200A while making the rupture strengths of the layers 310, 320, and 330 the same.

### <Example>

Next, an example of the catheter balloon disclosed here will be described. The scope of the catheter balloon disclosed here is not limited to the content described in the following example.

In the example, the following sample 1 (hereinafter, referred to as a "catheter balloon of the present example") and the following sample 2 (hereinafter, referred to as a "catheter balloon of a comparative example") were prepared.

### (Catheter Balloon of Present Example)

The catheter balloon of the present example was configured by a three-layer structure including the first layer 310, the second layer 320, and the third layer 330. Each of the layers 310, 320, and 330 has the same rupture strength of 50 MPa. Each of the layers 310, 320, and 330 has a film thickness of 37.6 µm, and an outer diameter Φ before dilation after blow molding is 5 mm.

### (Catheter Balloon of Comparative Example)

The catheter balloon of the comparative example was configured by the three-layer structure including the first layer 310, the second layer 320, and the third layer 330. A rupture strength of each of the first layer 310 and the third layer 330 is 18 MPa, and a rupture strength of the second layer 320 is 50 MPa. That is, the first layer 310 and the third layer 330 and the second layer 320 have different rupture strengths. Each of the layers 310, 320, and 330 has a film thickness of 37.6 µm, and an outer diameter Φ before dilation after blow molding is 5 mm.

### (Test Method)

Five "catheter balloons of the present example" described above and five" catheter balloons of the comparative example" described above were prepared. A pressurization device (fluid supply device) was connected to each catheter balloon, and a space portion was filled with liquid (water). The pressurization device was operated to increase a pressure in the space portion by 1 atm. At this time, a pressure holding time was set to 60 seconds. The pressure was increased as described above, and the pressure at which each catheter balloon ruptured was recorded as a rupture pressure. That is, pressure in the space portion was increased by 1 atm and then held for 60 seconds, followed by another 1 atm. pressure increase and a 60 second holding time, and future successive 1 atm. pressure increases separated by 60 second hold times until catheter balloon rupture.

From the above test, it was possible to confirm that the catheter balloon of the present example had a rupture pressure of 29.2 atm (average value of the five samples). On the other hand, it was possible to confirm that the catheter balloon of the comparative example had a rupture pressure of 26.0 atm (average value of the five samples). From this result, it was possible to confirm that the catheter balloon of the present example in which the rupture strengths of the layers 310, 320, and 330 were configured to be the same had higher pressure resistance than the catheter balloon of the comparative example.

The detailed description above describes an embodiment and modification of a catheter balloon representing examples of the new catheter balloon disclosed here. The invention is not limited, however, to the precise embodiment, modification and variations described. Various changes, modifications and equivalents can be effected by one skilled in the art without departing from the scope of the invention as defined in the accompanying claims. It is expressly intended that all such changes, modifications and equivalents that fall within the scope of the claims are embraced by the claims.

## Claims

1. A catheter balloon (200, 200A) comprising
a first layer (310) and a second layer (320) disposed adjacent to each other in a thickness direction of the balloon (200, 200A),
the first layer (310) and the second layer (320) being made of different materials, and
a rupture strength of the first layer (310) and a rupture strength of the second layer (320) being the same,
**characterized in that**,
the second layer (320) covers the first layer (310), and
an elongation percentage of the first layer (310) is larger than an elongation percentage of the second layer (320).

2. The catheter balloon (200, 200A) according to Claim 1, wherein hardness of the first layer (310) is smaller than hardness of the second layer (320).

3. The catheter balloon (200, 200A) according to Claim 2, wherein a tensile modulus of elasticity of the first layer (310) is smaller than a tensile modulus of elasticity of the second layer (320).

4. The catheter balloon (200, 200A) according to Claim 1, wherein
the first layer (310) is any one of a polyamide elastomer, a polyurethane, and a polyurethane elastomer, and
the second layer (320) is a polyamide.

5. The catheter balloon (200, 200A) according to Claim 1, further comprising
a third layer (330) disposed adjacent to the second layer (320) and covering the second layer (320),
wherein a rupture strength of the third layer (330) is the same as the rupture strength of the first layer (310) and the rupture strength of the second layer (320).

6. The catheter balloon (200, 200A) according to Claim 5, wherein
an elongation percentage of the first layer (310) and an elongation percentage of the third layer (330) are the same, and
the elongation percentage of the first layer (310) and the elongation percentage of the third layer (330) are larger than an elongation percentage of the second layer (320).

7. The catheter balloon (200, 200A) according to Claim 6, wherein
a hardness of the first layer (310) and hardness of the third layer (330) are the same, and
the hardness of the first layer (310) and the hardness of the third layer (330) are smaller than hardness of the second layer (320).

8. The catheter balloon (200, 200A) according to Claim 7, wherein
a tensile modulus of elasticity of the first layer (310) and a tensile modulus of elasticity of the third layer (330) are the same, and
the tensile modulus of elasticity of the first layer (310) and the tensile modulus of elasticity of the third layer (330) are smaller than a tensile modulus of elasticity of the second layer (320).

9. The catheter balloon (200, 200A) according to Claim 5, wherein
the first layer (310) is a polyamide elastomer,
the second layer (320) is a polyamide, and
the third layer (330) is a polyamide elastomer.

10. A catheter balloon (200, 200A) according to any claims 1 to 9, further connected to a shaft portion (100) and having an interior configured to receive a fluid to expand the catheter balloon (200, 200A), the catheter balloon (200, 200A) comprising:
a distal end portion and a proximal end portion at opposite ends of the catheter balloon (200, 200A), the distal end of the catheter balloon (200, 200A) being fixed to the shaft portion (100) and the proximal end of the catheter balloon (200, 200A) ballon being fixed to the shaft so that an interior of the catheter balloon (200, 200A) forms a space configured to receive a fluid to expand the catheter balloon (200, 200A);
the second layer (320) being positioned in covering relation to the first layer (310).

## Patentansprüche

1. Katheterballon (200, 200A), umfassend
eine erste Schicht (310) und eine zweite Schicht (320), die in einer Dickenrichtung des Ballons (200, 200A) benachbart zueinander angeordnet sind,
wobei die erste Schicht (310) und die zweite Schicht (320) aus unterschiedlichen Materialien hergestellt sind, und
wobei die Zerreißfestigkeit der ersten Schicht (310) und die Zerreißfestigkeit der zweiten Schicht (320) identisch sind,
**dadurch gekennzeichnet ist, dass**
die zweite Schicht (320) die erste Schicht (310) bedeckt, und
ein Prozentsatz der Dehnung der ersten Schicht (310) größer ist als ein Prozentsatz der Dehnung der zweiten Schicht (320).

2. Katheterballon (200, 200A) nach Anspruch 1, wobei die Härte von der ersten Schicht (310) geringer ist als die Härte der zweiten Schicht (320).

3. Katheterballon (200, 200A) nach Anspruch 2, wobei ein Zugelastizitätsmodul von der ersten Schicht (310) kleiner ist als ein Zugelastizitätsmodul der zweiten Schicht (320).

4. Katheterballon (200, 200A) nach Anspruch 1, wobei
die erste Schicht (310) irgendeine aus einem Polyamid-Elastomer, einem Polyurethan oder einem Polyurethan-Elastomer ist, und
die zweite Schicht (320) ein Polyamid ist.

5. Katheterballon (200, 200A) nach Anspruch 1, ferner umfassend
eine dritte Schicht (330), die benachbart zu der zweiten Schicht (320) angeordnet ist und die zweite Schicht (320) bedeckt,
wobei eine Zerreißfestigkeit der dritten Schicht (330) identisch ist mit der Zerreißfestigkeit der ersten Schicht (310) und mit der Zerreißfestigkeit der zweiten Schicht (320).

6. Katheterballon (200, 200A) nach Anspruch 5, wobei
ein Prozentsatz der Dehnung der ersten Schicht (310) und ein Prozentsatz der Dehnung der dritten Schicht (330) identisch sind, und
der Prozentsatz der Dehnung der ersten Schicht (310) und der Prozentsatz der Dehnung der dritten Schicht (330) größer sind als ein Prozentsatz der Dehnung der zweiten Schicht (320).

7. Katheterballon (200, 200A) nach Anspruch 6, wobei
eine Härte der ersten Schicht (310) und die Härte der dritten Schicht (330) identisch sind, und
die Härte von der ersten Schicht (310) und die Härte der dritten Schicht (330) geringer sind als die Härte der zweiten Schicht (320).

8. Katheterballon (200, 200A) nach Anspruch 7, wobei
ein Zugelastizitätsmodul von der ersten Schicht (310) und ein Zugelastizitätsmodul der dritten Schicht (330) identisch sind, und
der Zugelastizitätsmodul von der ersten Schicht (310) und der Zugelastizitätsmodul der dritten Schicht (330) kleiner sind als ein Zugelastizitätsmodul der zweiten Schicht (320).

9. Katheterballon (200, 200A) nach Anspruch 5, wobei die erste Schicht (310) ein Polyamid-Elastomer ist,
die zweite Schicht (320) ein Polyamid ist, und
die dritte Schicht (330) ein Polyamid-Elastomer ist.

10. Katheterballon (200, 200A) nach einem der Ansprüche 1 bis 9, der ferner mit einem Schaftabschnitt (100) verbunden ist und einen Innenraum aufweist, der so ausgebildet ist, dass er ein Fluid aufnimmt, um den Katheterballon (200, 200A) auszudehnen, wobei der Katheterballon (200, 200A) umfasst:
einen distalen Endabschnitt und einen proximalen Endabschnitt an gegenüberliegenden Enden des Katheterballons (200, 200A), wobei das distale Ende des Katheterballons (200, 200A) an dem Schaftabschnitt (100) befestigt ist und das proximale Ende des Katheterballons (200, 200A) an dem Schaft befestigt ist, sodass ein Innenraum des Katheterballons (200, 200A) einen Raum bildet, der so konfiguriert ist, dass er ein Fluid zur Ausdehnung des Katheterballons (200, 200A) aufnimmt;
wobei die zweite Schicht (320) in bedeckender Beziehung zu der ersten Schicht (310) angeordnet ist.

## Revendications

1. Ballonnet de cathéter (200, 200A) comprenant
une première couche (310) et une deuxième couche (320) disposées adjacentes l'une à l'autre dans une direction d'épaisseur du ballonnet (200, 200A),
la première couche (310) et la deuxième couche (320) étant constituées de matériaux différents, et
une résistance à la rupture de la première couche (310) et une résistance à la rupture de la deuxième couche (320) étant identiques,
**caractérisé en ce que**
la deuxième couche (320) recouvre la première couche (310), et
un pourcentage d'allongement de la première couche (310) est supérieur à un pourcentage d'allongement de la deuxième couche (320).

2. Ballonnet de cathéter (200, 200A) selon la revendication 1, dans lequel la dureté de la première couche (310) est inférieure à la dureté de la deuxième couche (320).

3. Ballonnet de cathéter (200, 200A) selon la revendication 2, dans lequel un module d'élasticité en traction de la première couche (310) est plus petit qu'un module d'élasticité en traction de la deuxième couche (320).

4. Ballonnet de cathéter (200, 200) selon la revendication 1, dans lequel
la première couche (310) est l'un quelconque d'un élastomère de polyamide, d'un polyuréthane et d'un élastomère de polyuréthane, et
la deuxième couche (320) est un polyamide.

5. Ballonnet de cathéter (200, 200A) selon la revendication 1, comprenant en outre
une troisième couche (330) disposée adjacente à la deuxième couche (320) et recouvrant la deuxième couche (320),
dans lequel une résistance à la rupture de la troisième couche (330) est identique à la résistance à la rupture de la première couche (310) et à la résistance à la rupture de la deuxième couche (320).

6. Ballonnet de cathéter (200, 200A) selon la revendication 5, dans lequel
un pourcentage d'allongement de la première couche (310) et un pourcentage d'allongement de la troisième couche (330) sont identiques, et
le pourcentage d'allongement de la première couche (310) et le pourcentage d'allongement de la troisième couche (330) sont supérieurs à un pourcentage d'allongement de la deuxième couche (320).

7. Ballonnet de cathéter (200, 200A) selon la revendication 6, dans lequel
une dureté de la première couche (310) et une dureté de la troisième couche (330) sont identiques, et
la dureté de la première couche (310) et la dureté de la troisième couche (330) sont inférieures à la dureté de la deuxième couche (320).

8. Ballonnet de cathéter (200, 200A) selon la revendication 7, dans lequel
un module d'élasticité en traction de la première couche (310) et un module d'élasticité en traction de la troisième couche (330) sont identiques, et
le module d'élasticité en traction de la première couche (310) et le module d'élasticité en traction de la troisième couche (330) sont inférieurs à un module d'élasticité en traction de la deuxième couche (320).

9. Ballonnet de cathéter (200, 200A) selon la revendication 5, dans lequel
la première couche (310) est un élastomère de polyamide, la deuxième couche (320) est un polyamide, et
la troisième couche (330) est un élastomère de polyamide.

10. Ballonnet de cathéter (200, 200A) selon l'une quelconque des revendications 1 à 9, en outre relié à une partie tige (100) et ayant un intérieur configuré pour recevoir un fluide pour dilater le ballonnet de cathéter (200, 200A), le ballonnet de cathéter (200, 200A) comprenant :
une partie d'extrémité distale et une partie d'extrémité proximale au niveau d'extrémités opposées du ballonnet de cathéter (200, 200A), l'extrémité distale du ballonnet de cathéter (200, 200A) étant fixée à la partie tige (100) et
l'extrémité proximale du ballonnet de cathéter (200, 200A) étant fixée à la tige de sorte qu'un intérieur du ballonnet de cathéter (200, 200A) forme un espace configuré pour recevoir un fluide pour dilater le ballonnet de cathéter (200, 200A) ;
la deuxième couche (320) étant positionnée en relation de recouvrement par rapport à la première couche (310).
